# EUROPEAN PATENT SPECIFICATION

(11) **EP 4 611 881 B1**
(45) Date of publication and mention of the grant of the patent: **25.11.2026**
(21) Application number: 23813942.2
(22) Date of filing: 30.10.2023
(51) Int. Cl.: A61N 1/375

(54) **IMPLANTABLE DEVICE ENCLOSURES**
GEHÄUSE FÜR IMPLANTIERBARE GERÄTE
SUPPORTS D'APPAREILS IMPLANTABLES

(30) Priority: 04.11.2022 US 202263422689 P
(43) Date of publication of application: 10.09.2025
(60) Divisional application: 26213286.3
(73) Proprietor: TC1 LLC, St. Paul, MN 55117 (US)
(72) Inventor: IUDICE, Jeffrey, St. Paul, Minnesota 55117 (US); HANSEN, John Freddy, St. Paul, Minnesota 55117 (US); BOBBA, Veera Venkata Jagadeesh, St. Paul, Minnesota 55117 (US); ANDERSON, Russell, St. Paul, Minnesota 55117 (US); RAPOSO, Luann, St. Paul, Minnesota 55117 (US); TRUONG, Soy, St. Paul, Minnesota 55117 (US); LUY, Chanthy, St. Paul, Minnesota 55117 (US); ELLEDGE, Jason, St. Paul, Minnesota 55117 (US); SYLVESTER, JR., Joseph P., St. Paul, Minnesota 55117 (US); NGUYEN, John Hai, St. Paul, Minnesota 55117 (US); BAVAL, Alexander, St. Paul, Minnesota 55117 (US); LEPE, Jose, St. Paul, Minnesota 55117 (US)
(74) Representative: Deambrogi, Edgardo
(86) International application number: PCT/US2023/036270
(87) International publication number: WO 2024/097127

(56) References cited:
- CN-A- 102 614 580
- US-A- 5 782 891
- US-A1- 2013 331 923
- US-A1- 2018 071 538
- US-A1- 2018 197 661
- US-B1- 7 447 533
- US-B2- 8 253 555
- US-B2- 9 398 901
- US-B2- 9 620 293

## Description

### BACKGROUND OF THE DISCLOSURE

### a. Field of the Disclosure

The present disclosure relates generally to implantable devices, and more specifically, relates to enclosures for implantable devices.

### b. Background

There are various types of implantable devices, including implantable medical devices with active electronics. To protect a subject in which an electronic device is implanted, and to ensure proper operation of such a device with active electronics, the device typically includes a housing that encloses one or more components of the electronics in the device. The housing is also typically hermetically sealed.

In at least some known systems, such housings are metallic (e.g., titanium). However, metallic housings may have undesirable thermal and/or electrical properties. For example, at least some known systems use a wireless power transmitter and receiver to wirelessly transmit power to an implanted device (e.g., a ventricular assist device). In such systems, it may be desirable to reduce the amount of metal used (e.g., to reduce interference with operation of the system). Further, some subjects may be allergic to certain metals used in known metallic housings.

Accordingly, it would be desirable to provide an alternative housing for implantable devices. An implantable medical device with a ceramic enclosure is disclosed in US 5,782,891.

### SUMMARY OF THE DISCLOSURE

In one aspect, an enclosure for an implantable device is provided. The enclosure includes a cup defining a cavity, the cup including a non-metallic body, and a weld ring coupled to the non-metallic body, and a lid, wherein the lid is weldable to the weld ring to couple the cup to the lid and hermetically seal the enclosure.

In another aspect, an implantable device assembly is provided. The implantable device assembly includes an enclosure including a cup defining a cavity, the cup including a non-metallic body, and a weld ring coupled to the non-metallic body, and a lid welded to the weld ring to couple the cup to the lid and hermetically seal the enclosure. The implantable device assembly further includes an implantable device positioned within the cavity of the enclosure.

In yet another aspect, a method of assembling an enclosure for an implantable device is provided. The method includes coupling a non-metallic body to a weld ring to form a cup, the cup defining a cavity, forming a lid, and welding lid to the weld ring to couple the cup to the lid and hermetically seal the enclosure.

In the following, aspects, embodiments, examples and methods of the present disclosure that do not fall within the scope of the appended claims are provided for illustrative purposes.

### BRIEF DESCRIPTION OF THE DRAWINGS

FIG. 1 is a simplified electrical circuit diagram of one embodiment of a wireless power transfer system.
FIG. 2 is an illustration of the wireless power transfer system of FIG. 1 being used to supply power to a ventricular assist device (VAD).
FIG. 3 is a front perspective view of components of one embodiment of a wireless power transfer system.
FIG. 4 is a perspective view of one embodiment of an enclosure for housing an implantable device.
FIG. 5 is an exploded view of the enclosure show in FIG. 4 and an implantable device.
FIG. 6 is a cross-sectional view of a portion of the enclosure shown in FIG. 4 taken along line 6-6 (shown in FIG. 4).
FIGS. 7 and 8 are perspective views of one embodiment of a welding assembly that may be used to weld the cup to the lid of the enclosure shown in FIG. 4.

### DETAILED DESCRIPTION OF THE DISCLOSURE

The present disclosure is directed to an enclosure for an implantable device. The enclosure includes a cup defining a cavity, the cup including a non-metallic body, and a weld ring coupled to the non-metallic body, and a lid, wherein the lid is weldable to the weld ring to couple the cup to the lid and hermetically seal the enclosure.

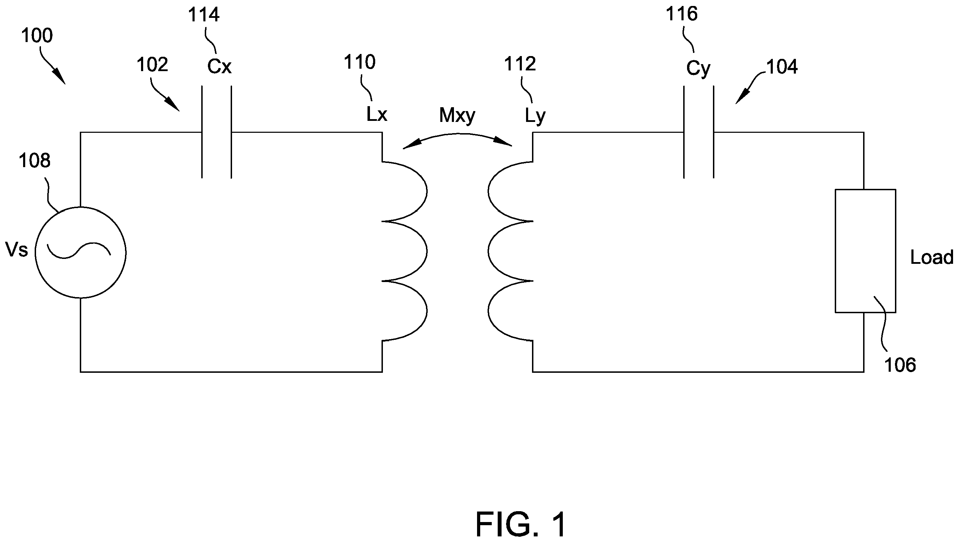

Referring now to the drawings, FIG. 1 is a simplified circuit of an example wireless power transfer system 100. The system 100 includes an external transmit resonator 102 and an implantable receive resonator 104. In the system shown in FIG. 1, a power source Vs is electrically connected with the transmit resonator 102, providing power to the transmit resonator 102. The receive resonator 104 is connected to a load 106 (e.g., an implantable medical device). The receive resonator 104 and the load 106 may be electrically connected with a switching or rectifying device (not shown).

In the example embodiment, the transmit resonator 102 includes a coil Lx connected to the power source Vs by a capacitor Cx. Further, the receive resonator 104 includes a coil Ly connected to the load 106 by a capacitor Cy. Inductors Lx and Ly are coupled by a coupling coefficient k. M_{xy} is the mutual inductance between the two coils. The mutual inductance, M_{xy}, is related to the coupling coefficient k as shown in the below Equation (1). ${M}_{xy} = k \sqrt{{L}_{x} ⋅ {L}_{y}}$ (1)

In operation, the transmit resonator 102 transmits wireless power received from the power source Vs. The receive resonator 104 receives the power wirelessly transmitted by the transmit resonator 102, and transmits the received power to the load 106.

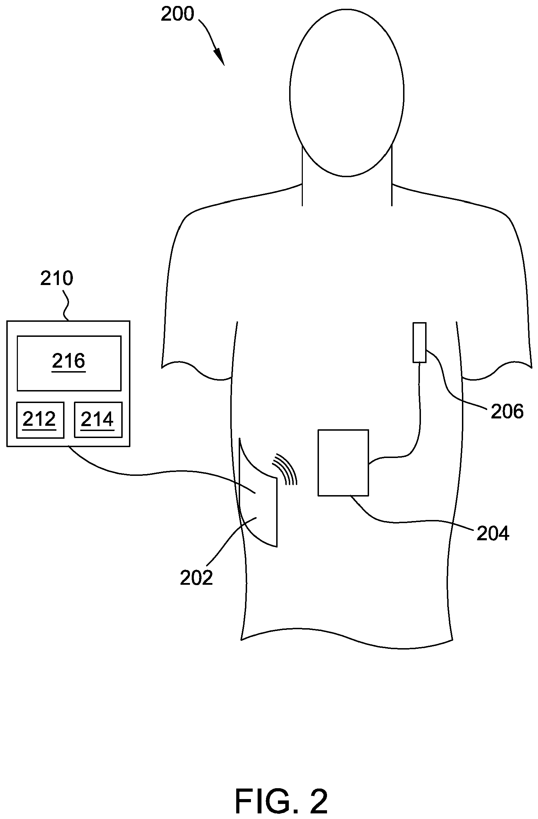

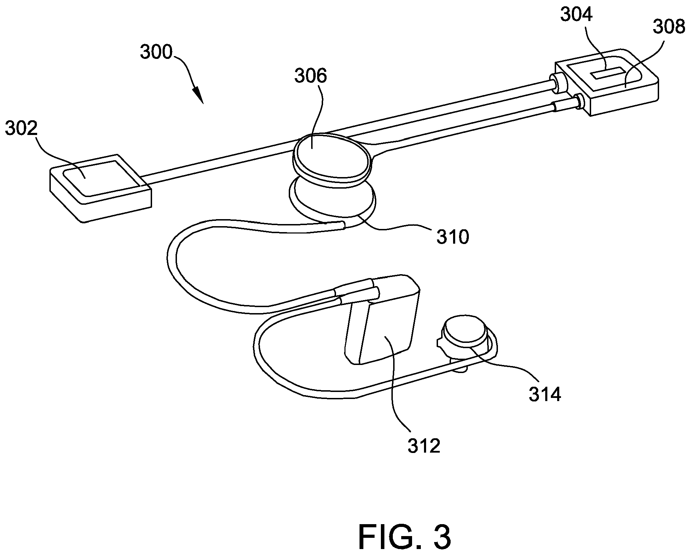

FIG. 2 illustrates one embodiment of a patient 200 using an external coil 202 (such as the transmit resonator 102 shown in FIG. 1) to wirelessly transmit power to an implanted coil 204 (such as the receive resonator shown in FIG. 1). The implanted coil 204 uses the received power to power an implanted device 206. For example, the implanted device 206 may include a pacemaker or heart pump (e.g., a left ventricular assist device (LVAD)). In some embodiments, the implanted coil 204 and/or the implanted device 206 may include or be coupled to a controller or a battery. For example, in some embodiments, a controller and/or a battery may be coupled between the implanted coil 204 and the implanted device 206 (see, e.g., FIG. 3).

In one embodiment, the external coil 202 is communicatively coupled to a computing device 210, for example, via wired or wireless connection, such that the external coil 202 may receive signals from and transmit signals to the computing device 210. In some embodiments, the computing device 210 is a power source for the external coil 202. In other embodiments, the external coil 202 is coupled to an alternative power supply (not shown). The computing device 210 includes a processor 212 in communication with a memory 214. In some embodiments, executable instructions are stored in the memory 214.

The computing device 210 further includes a user interface (UI) 216. The UI 216 presents information to a user (e.g., the patient 200). For example, the UI 216 may include a display adapter (not shown) that may be coupled to a display device, such as a cathode ray tube (CRT), a liquid crystal display (LCD), an organic LED (OLED) display, and/or an "electronic ink" display. In some embodiments, the UI 216 includes one or more display devices. Further, in some embodiments, presentation interface may not generate visual content, but may be limited to generating audible and/or computer-generated spoken-word content. In the example embodiment, the UI 216 displays one or more representations designed to aid the patient 200 in placing the external coil 202 such that the coupling between the external coil 202 and the implanted coil 204 is optimal. In some embodiments, the computing device 210 may be a wearable device. For example, in one embodiment, the computing device 210 is a wrist watch, and the UI 216 is displayed on the wrist watch.

FIG. 3 is a perspective view of components of one embodiment of a wireless power transfer system 300. System includes a battery pack 302, a hub device 304 (e.g., the computing device 210 shown in FIG. 2), and a transmitter 306 (e.g., the external coil 202 shown in FIG. 2). The hub device 304 is coupled between the battery pack 302 and the transmitter 306. Further, the hub device 304 includes a user interface (UI) 308 that enables a user to operate the wireless power transfer system 300. For example, the user may control the amount of power supplied from the battery pack 302 to the transmitter 306 using the UI 308. The wireless power transfer system 300 further includes an implanted receiver 310 (e.g., the implanted coil 204 shown in FIG. 2), a controller 312, and an implanted device 314 (e.g., the implanted device 206 shown in FIG. 2). The implanted device 314 may be, for example, a heart pump. The controller 312 is coupled between the implanted device 314 and the receiver 310. The controller 312 controls operation of the implanted device 314 (e.g., by controlling power delivery from the receiver 310 to the implanted device 314).

The battery pack 302, the hub device 304, and the transmitter 306 are located outside a patient's body (e.g., the patient 200, shown in FIG. 2) during operation of the wireless power transfer system 300. In contrast, the receiver 310, the controller 312, and the implanted device 314 are implanted within the patient's body during operation of the wireless power transfer system 300. During operation, the transmitter 306 wirelessly transmits radio-frequency (RF) power to the receiver 310.

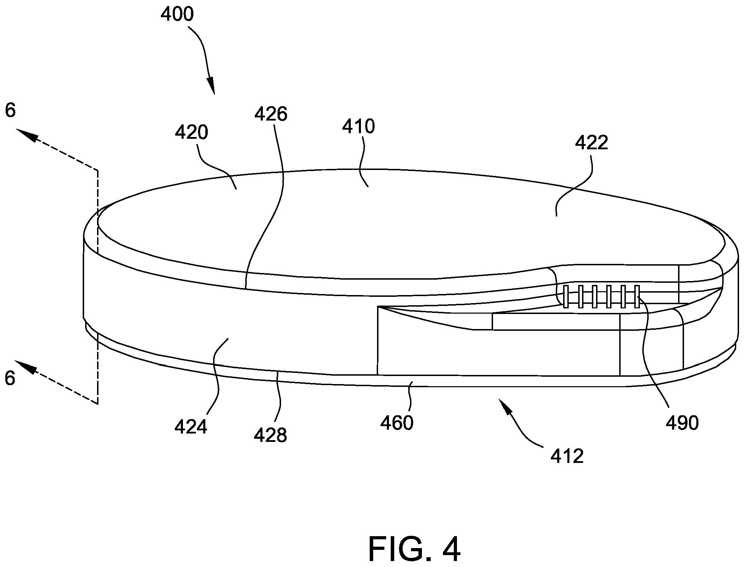

FIG. 4 is a perspective view of one embodiment of an enclosure 400 for housing an implantable device with active electronics. For example, the receiver 310 (shown in FIG. 3) may include the enclosure 400 with a wireless power resonator contained therein.

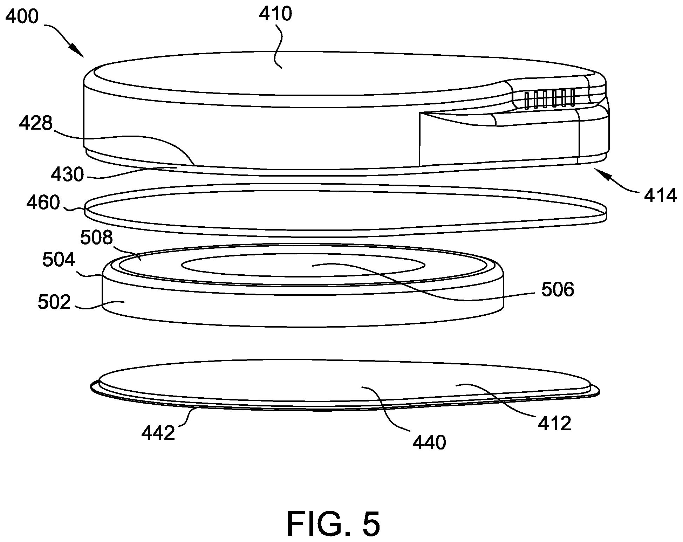

FIG. 5 is an exploded view of the enclosure 400 and an implantable device 502. In the embodiment of FIG. 5, the implantable device 502 is a wireless power resonator 504 including a core 506 and a coil element 508.

The core 506 is formed of a magnetic material, and may, for example, be formed of a ferrite material, such as nickel-based or manganese-based ferrites. Nickel-based ferrites generally have lower electrical conductivity and reduced losses, while manganese-based ferrites have a higher magnetic permeability (while still having acceptable losses), facilitating containing magnetic field lines, and reducing fringing fields entering nearby conductors (e.g., a titanium enclosure or copper in a nearby PCB) to prevent losses. In other embodiments, other types of ferrite materials may be used. For example, in some embodiments, a magnesium-based ferrite (e.g., MgCuZn, which may outperform nickel-based and manganese-based ferrites in a frequency range around 1 Megahertz (MHz)) may be used.

The wireless power resonator 504 may be, for example, a Litz wire resonator or a stacked plate resonator. In a Litz wire resonator, the coil element 508 includes a plurality of loops of Litz wire. In a stacked plate resonator, the coil element 508 includes a plurality of stacked plates that may include a plurality of alternating dielectric layers and conductive layers arranged in a stack. The dielectric layers may be formed of, for example, ceramic, plastic, glass, and/or mica.

Although the implantable device 502 is a wireless power resonator 504 in the embodiment shown in FIG. 5, those of skill in the art will appreciate that the enclosure 400 may be used to house any suitable implantable device.

In the embodiment shown, the enclosure 400 is a non-metallic hermetic enclosure, which has several advantages as compared to a metallic housing. For example, fabricating the enclosure 400 from a non-metallic material and using the enclosure for a wireless power receiver (such as receiver 310 (shown in FIG. 3)) reduces the thermal resistance of the receiver, which in turn reduces interference with the RF power transmission, reduces heating, and reduces RF emissions. In addition, the non-metallic enclosure 400 is electrically and thermodynamically energy efficient. The non-metallic enclosure 400 creates a hermetic barrier for active implantable devices by using a ceramic alternative (e.g., alumina) rather than a metal (e.g., titanium) to prevent moisture from comprising electronics housed therein. The non-metallic material of the enclosure 400 is also advantageous in other long-term active implantable applications where a metal enclosure may not be feasible (e.g., for patients who have an allergic reaction to metal implants).

The enclosure 400 includes a cup 410 and a lid 412. In the embodiment shown, the cup 410 is a three-dimensional bowl or boxed-shaped structure that defines a cavity 414. The cup 410 includes a non-metallic (e.g., ceramic) body 420 having an end wall 422 and a side wall 424 extending generally perpendicular to the end wall 422. Specifically, the side wall 424 extends from a first end 426 proximate the end wall 422 to a second end 428 proximate an opening of the cavity 414.

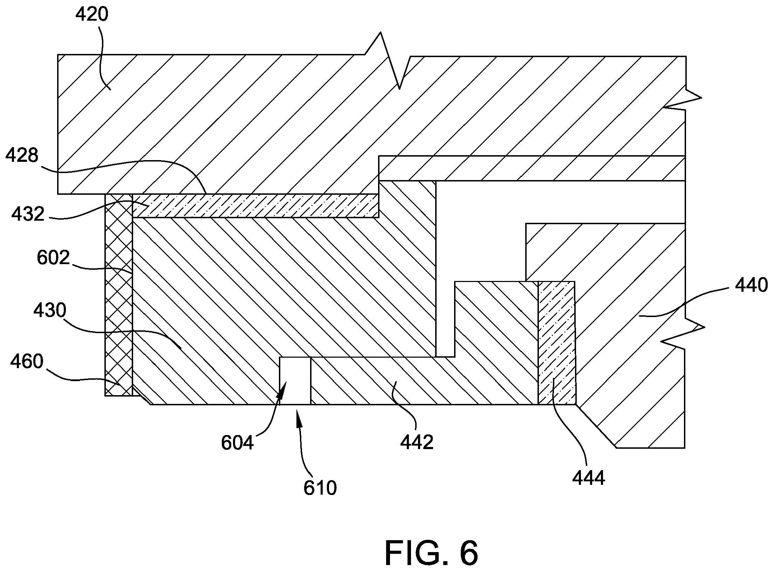

A first weld ring 430 is coupled to the second end 428 of the side wall 424 via a first braze 432 (shown in FIG. 6). Specifically, the first braze 432 joins the first weld ring 430 to the non-metallic body 420 and creates a hermetic barrier between the first weld ring 430 and the non-metallic body 420. The first braze 432 may be, for example, gold, and the first weld ring 430 may be, for example, titanium. The first weld ring 430 defines a perimeter of the cavity 414, and facilitates welding the lid 412 to the cup 410, as explained in further detail below.

In this embodiment, the lid 412 includes a non-metallic (e.g., ceramic) base 440 coupled to a second weld ring 442 via a second braze 444 (shown in FIG. 6). Specifically, the second braze 444 joins the second weld ring 442 to the non-metallic base 440 and creates a hermetic barrier between the second weld ring 442 and the non-metallic base 440. The second braze 444 may be, for example, gold, and the second weld ring 442 may be, for example, titanium. The second weld ring 442 defines a perimeter of the lid 412.

In the embodiment shown, the non-metallic base 440 is a plate-shaped component. Alternatively, the non-metallic base 440 may be bowl or box-shaped, similar to the shape of the non-metallic body 420 of the cup 410. Further, in some embodiments, a metal plate (not shown), such as a copper plate, may be coupled to the non-metallic base 440. The metal plate may be coupled to an outer surface of the non-metallic base 440 (i.e., facing away from the cup 410) to facilitate spreading heat generated by components housed in the enclosure 400.

In an alternative embodiment, the entire lid is metal. For example, the lid may be titanium. In such an embodiment, because the lid is metal, the second weld ring 442 and the second braze 444 are not included. Instead, the lid includes a metal base.

In some embodiments, a metal ring 460 is coupled to the cup 410. Specifically, the metal ring 460 is coupled to the first weld ring 430 (e.g., using a medical grade adhesive). The metal ring 460 is a relatively thin strip of material (e.g., having a thickness of approximately 0.1 millimeters (mm), 0.2 mm, or 0.5 mm) and may be made of a material with a relatively high electrical conductivity. For example, the metal ring 460 may be silver. Alternatively, the metal ring 460 may be made of another material in other embodiments (e.g., copper, aluminum, and/or gold). The metal ring 460 facilitates further improving RF and thermal performance of the enclosure 400.

FIG. 6 is a cross-sectional view of a portion of the enclosure 400 taken along line 6-6 (shown in FIG. 4). Specifically, FIG. 6 illustrates an example arrangement between the non-metallic body 420, the first weld ring 430, the first braze 432, the non-metallic base 440, the second weld ring 442, the second braze 444, and the metal ring 460.

As shown in FIG. 6, the first braze 432 couples the non-metallic body to 420 to the first weld ring 430. Further, the second braze 444 couples the nonmetallic base 440 to the second weld ring 442. In addition, as shown in FIG. 6, the metal ring 460 is attached to an outer surface 602 of the first weld ring 430.

To seal electronics and other components (e.g., the wireless power resonator 504) within the enclosure 400, the lid 412 is positioned to cover the cavity 414 of the cup 410. In this configuration, the first weld ring 430 and the second weld ring 442 meet and form a weld seam 604. The first weld ring 430 and the second weld ring 442 are welded together at the weld seam 604, creating a hermetic seal between the cup 410 and the lid 412. In the alternative embodiment where the lid is metal, the metal base of the lid may be welded directly to the first weld ring 430 (omitting the second weld ring 442 and the second braze 444 as noted above).

The shape, location, and orientation of the weld seam 604 facilitates avoiding heat transfer to the first braze 432 and the second braze 444 during welding. Further, the weld seam 604 has a relatively low profile. The geometry of the weld seam 604 also maintains relatively small weld gaps 610 between the components, facilitating laser welding at low power to avoid impacting the first braze 432 and the second braze 444. Accordingly, the enclosure 400 provides a hermetic hybrid assembly (e.g., metal and non-metal) with tight tolerances that allow for low power welding.

The materials used for the non-metallic body 420, the first weld ring 430, the first braze 432, the non-metallic base 440, the second weld ring 442, the second braze 444, and the metal ring 460 are selected, based at least in part, on matching the coefficients of thermal expansions of the different components to facilitate minimizing residual stress during heating and cooling. In one example, the non-metallic body 420 and the non-metallic base 440 are alumina, the first weld ring 430 and the second weld ring 442 are titanium, the first braze 432 and the second braze 444 are gold, and the metal ring 460 is silver. Alternatively, any suitable materials may be used.

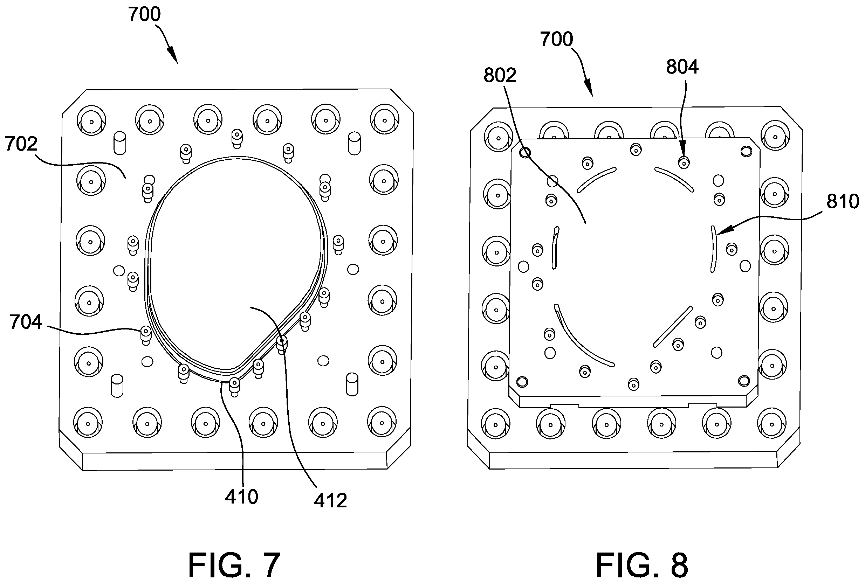

FIGS. 7 and 8 are perspective views of one embodiment of a welding assembly 700 that may be used to weld the cup 410 to the lid 412 to seal the enclosure 400. The welding assembly 700 includes a nest 702 and a plurality of pins 704. To perform the welding, the cup 410 and the lid 412 are placed in the nest 702. Then, as shown in FIG. 8, a guide plate 802 is positioned over the cup and the lid 412. The guide plate 802 includes a plurality of holes 804 that receive corresponding pins 704 to align the guide plate 802 with the cup 410 and the lid 412.

The welding assembly 700 securely clamps the cup 410 and the lid 412 together. Welding is performed at each of a plurality of welding windows 810 defined in the guide plate 802. Then, the guide plate 802 may be removed, and gaps between the individual weld spots are filled by performing additional welding. Alternatively, the cup 410 and the lid 412 may be welded together using any suitable technique.

Referring back to FIG. 4, in the embodiment shown, a plurality of feedthrough pins 490 extend through the cup 410. The feedthrough pins 490 facilitate electrically and/or communicatively coupling components within the enclosure 400 to devices external to the enclosure 400. The feedthrough pins 490 may be, for example, platinum-iridium.

For a metallic housing (e.g., in at least some known systems), feedthrough pins must be electrically isolated from the housing. For example, a ceramic substrate may be used to isolate the feedthrough pins from the housing. In the present disclosure, however, because the enclosure 400 is non-metallic (and electrically non-conductive), no additional materials are needed. Accordingly, the feedthrough pins 490 can be inserted through the cup 410 and brazed directly to the cup 410 (e.g., using a gold braze) to secure them in place, reducing manufacturing complexity and costs.

The embodiments described herein are directed an enclosure for an implantable device. The enclosure includes a cup defining a cavity, the cup including a non-metallic body, and a weld ring coupled to the non-metallic body, and a lid, wherein the lid is weldable to the weld ring to couple the cup to the lid and hermetically seal the enclosure.

Although the embodiments and examples disclosed herein have been described with reference to particular embodiments, it is to be understood that these embodiments and examples are merely illustrative of the principles and applications of the present disclosure. It is therefore to be understood that numerous modifications can be made to the illustrative embodiments and examples and that other arrangements can be devised without departing from the scope of the present disclosure as defined by the claims. Thus, it is intended that the present application cover the modifications and variations of these embodiments and their equivalents.

This written description uses examples to disclose the disclosure, including the best mode, and also to enable any person skilled in the art to practice the disclosure, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An enclosure (400) for an implantable device (502), the enclosure comprising:
a cup (410) defining a cavity (414), the cup comprising:
a non-metallic body(420) extending between an end wall (422) and a second end (428); and
a weld ring (430) coupled to the second end of the non-metallic body, the weld ring defining an outer surface (602); and
a lid (412), wherein the lid is weldable to the weld ring to couple the cup to the lid and hermetically seal the enclosure; and
a metal ring (460) coupled to the outer surface of the weld ring.

2. The enclosure of claim 1, wherein the lid comprises:
a non-metallic base (440); and
a lid wed ring (442) coupled to the non-metallic base, wherein the weld ring is weldable to the lid weld ring to couple the cup to the lid and hermetically seal the enclosure.

3. The enclosure of claim 2, wherein the non-metallic base is coupled to the lid ring via a braze.

4. The enclosure of claim 1, wherein the lid is metal.

5. The enclosure of claim 1, wherein the non-metallic body is coupled to the weld ring via a braze.

6. The enclosure of claim 1, wherein the metal ring is silver.

7. The enclosure of claim 1, further comprising a plurality of feedthrough pins (490) extending through the cup and brazed to the cup.

8. The enclosure of claim 1, wherein the non-metallic body is ceramic.

9. The enclosure of claim 1, wherein the weld ring is titanium.

10. An implantable device assembly comprising:
the enclosure of claim 1; and
an implantable device (502) positioned within the cavity of the enclosure.

11. The implantable device assembly of claim 10, wherein the implantable device is a wireless power resonator (504).

## Patentansprüche

1. Gehäuse (400) für eine implantierbare Vorrichtung (502), wobei das Gehäuse Folgendes umfasst:
einen Becher (410), der einen Hohlraum (414) definiert, wobei der Becher Folgendes umfasst:
einen nichtmetallischen Körper (420), der sich zwischen einer Endwand (422) und einem zweiten Ende (428) erstreckt; und
einen Schweißring (430), der mit dem zweiten Ende des nichtmetallischen Körpers gekoppelt ist, wobei der Schweißring eine Außenfläche (602) definiert; und
einen Deckel (412), wobei der Deckel mit dem Schweißring verschweißbar ist, um den Becher mit dem Deckel zu koppeln und das Gehäuse hermetisch abzudichten; und
einen Metallring (460), der mit der Außenfläche des Schweißrings gekoppelt ist.

2. Gehäuse nach Anspruch 1, wobei der Deckel Folgendes umfasst:
eine nichtmetallische Basis (440); und
einen Deckelschweißring (442), der mit der nichtmetallischen Basis gekoppelt ist, wobei der Schweißring mit dem Deckelschweißring verschweißbar ist, um den Becher mit dem Deckel zu koppeln und das Gehäuse hermetisch abzudichten.

3. Gehäuse nach Anspruch 2, wobei die nichtmetallische Basis mittels einer Hartlötverbindung mit dem Deckelring gekoppelt ist.

4. Gehäuse nach Anspruch 1, wobei der Deckel aus Metall gebildet ist.

5. Gehäuse nach Anspruch 1, wobei der nichtmetallische Körper mittels einer Hartlötverbindung mit dem Schweißring gekoppelt ist.

6. Gehäuse nach Anspruch 1, wobei der Metallring aus Silber gebildet ist.

7. Gehäuse nach Anspruch 1, ferner umfassend eine Vielzahl von Durchführungsstiften (490), die sich durch den Becher erstrecken und mit dem Becher hartverlötet sind.

8. Gehäuse nach Anspruch 1, wobei der nichtmetallische Körper aus Keramik gebildet ist.

9. Gehäuse nach Anspruch 1, wobei der Schweißring aus Titan gebildet ist.

10. Implantierbare Vorrichtungsanordnung, umfassend:
das Gehäuse nach Anspruch 1; und
eine implantierbare Vorrichtung (502), die innerhalb des Hohlraums des Gehäuses angeordnet ist.

11. Implantierbare Vorrichtungsanordnung nach Anspruch 10, wobei die implantierbare Vorrichtung ein drahtloser Leistungsresonator (504) ist.

## Revendications

1. Un support (400) pour un appareil implantable (502), le support comprenant :
une coupelle (410) définissant une cavité (414), la coupelle comprenant :
un corps non métallique (420) s'étendant entre une paroi d'extrémité (422) et une seconde extrémité (428) ; et
un anneau de soudure (430) couplé à la seconde extrémité du corps non métallique, l'anneau de soudure définissant une surface extérieure (602) ; et
un couvercle (412), dans lequel le couvercle est soudable à l'anneau de soudure pour coupler la coupelle au couvercle et sceller hermétiquement le support ; et
un anneau métallique (460) couplé à la surface extérieure de l'anneau de soudure.

2. Le support selon la revendication 1, dans lequel le couvercle comprend :
une base non métallique (440) ; et
un anneau de soudure de couvercle (442) couplé à la base non métallique, dans lequel l'anneau de soudure est soudable à l'anneau de soudure de couvercle pour coupler la coupelle au couvercle et sceller hermétiquement le support.

3. Le support selon la revendication 2, dans lequel la base non métallique est couplée à l'anneau de couvercle par brasage.

4. Le support selon la revendication 1, dans lequel le couvercle est métallique.

5. Le support selon la revendication 1, dans lequel le corps non métallique est couplé à l'anneau de soudure par brasage.

6. Le support selon la revendication 1, dans lequel l'anneau métallique est en argent.

7. Le support selon la revendication 1, comprenant en outre une pluralité de broches de traversée (490) s'étendant à travers la coupelle et brasées à la coupelle.

8. Le support selon la revendication 1, dans lequel le corps non métallique est en céramique.

9. Le support selon la revendication 1, dans lequel l'anneau de soudure est en titane.

10. Un ensemble d'appareil implantable comprenant :
le support selon la revendication 1 ; et
un appareil implantable (502) positionné à l'intérieur de la cavité du support.

11. L'ensemble d'appareil implantable selon la revendication 10, dans lequel l'appareil implantable est un résonateur de puissance sans fil (504).
